# EUROPÄISCHE PATENTSCHRIFT

(11) **EP 1 885 293 B1**
(45) Veröffentlichungstag und Bekanntmachung des Hinweises auf die Patenterteilung: **28.04.2010**
(21) Anmeldenummer: 06701286.4
(22) Anmeldetag: 12.01.2006
(51) Int. Cl.: A61F 2/36, A61F 2/34

(54) **SET ZUR ERSTELLUNG EINES RESURFACING-HÜFTIMPLANTATES**
SET FOR PRODUCING A RESURFACING HIP IMPLANT
ENSEMBLE POUR LA CREATION D'UN IMPLANT DE RESURFAÇAGE DE LA HANCHE

(30) Priorität: 04.03.2005 DE 102005011361
(43) Veröffentlichungstag der Anmeldung: 13.02.2008
(73) Patentinhaber: ESKA Implants GmbH & Co. KG, 23556 Lübeck (DE)
(72) Erfinder: GRUNDEI, Hans, 23558 Lübeck (DE); GERDESMEYER, Ludger, 24105 Kiel (DE)
(74) Vertreter: Fuchs
(86) Internationale Anmeldenummer: PCT/EP2006/000212
(87) Internationale Veröffentlichungsnummer: WO 2006/094572

(56) Entgegenhaltungen:
- EP-A- 0 339 816
- DE-U1- 9 402 828
- DE-U1- 29 824 009
- US-A- 4 035 848
- US-A- 4 173 797
- US-A1- 2003 171 818
- US-A1- 2003 171 820

## Beschreibung

Die Erfindung betrifft ein Set zur Erstellung eines Resurfacing-Hüftimplantates. Hierunter wird ein Oberflächenersatz für die natürlichen Gleit- oder Artikulationsflächen des Acetabulums und des Hüftgelenkskopfes verstanden.

In jüngster Zeit kommen verstärkt so genannte Kappenimplantate zur Anwendung, welche über den präparierten natürlichen Restgelenkkopf des Hüftgelenkes gesetzt werden und in dieser Lage dann fixiert werden können. Kappenimplantate bestehen aus einer der äußeren Form der natürlichen Gelenkkugel nachgebildeten Kappe, die auf einen (teil-)präparierten natürlichen Restgelenkkopf setzbar ist. Ein derartiges Implantat lässt sich aus dem so genannten Set zur Erstellung eines Armierungsimplantates gemäß der DE-C-102 18 801 erstellen.

Voraussetzung für eine stabile Sekundärfixation ist stabiles Knochenmaterial des Restknochens. So wird in der schon erwähnten Druckschrift vorgeschlagen, an die Gelenkkopfkappe einen Zapfen anzukoppeln, der in eine entsprechende Ausfräsung im Schenkelhals gesetzt wird. Dieser Zapfen weist eine Oberfläche auf, welche mit einer dreidimensionalen offenmaschigen Raumnetzstruktur versehen ist, in welche und durch welche hindurch Knochentrabekel des umliegenden Knochenmaterials wachsen und für die stabile Sekundärfixation sorgen.

Es gibt jedoch Indikationen, bei denen man noch davon absehen kann, den Schenkelhals auszufräsen, um so Platz für den Zapfen zu schaffen. Angeführt wird hier die sogenannte Perthes-Calve-Legg-Krankheit, die ein- oder beidseitig im Bereich der Femurkopfepiphyse aseptisch Knochennekrosen hervorruft. Vor allem bei Jungen vom 4. bis 12. Lebensjahr tritt diese Krankheit auf (Pschyrembel, Klinisches Wörterbuch, 259. Auflage, 2002, Seite 1285). Eine Ausheilung ohne Deformierung ist zwar möglich, jedoch bleibt eine eventuelle Walzen- oder Pilzform des Schenkelkopfes mit Abplattung der Hüftgelenkpfanne, seltener Coxa Plana oder Arthrosis deformans zurück.

Eine weitere Indikation ist beispielsweise eine Zyste im Hüftgelenkskopf, die zu Oberflächendefekten des Gelenkkopfes führt.

Ganz generell kann eine Nekrose des Gelenkkopfes zu oberflächenhaften Defekten führen, die es aber immer noch nicht rechtfertigen, den Gelenkkopf vollständig zu resezieren und den Patienten mit einer Kurzstielendoprbthese (EP 0 878176) zu versorgen.

Grundsätzlich - und dies wurde in letzter Zeit verstärkt erkannt - ist es günstig, mit (Teil-)Resektionen von Knochen solange wie möglich zu zögern, um bei einem eventuell später notwendigen Revisionseingriff auf mehrere Stufen der endoprothetischen Versorgung zurückgreifen zu können, von der Kurzstielendoprothese bis zur Langstielendoprothese. Der Einsatz der letztgenannten Endoprothese erfordert die vollständige Resektion des Schenkelhalses.

Eine künstliche Hüftprothese ist bekannt geworden aus US-A-4 173 797. Allerdings findet bei der Implantation dieses Gelenks auch die Anwendung von Formfräsern statt, so dass die beteiligten natürlichen Knochen in Mitleidenschaft gezogen werden insofern, als dass sie deutlich angefrischt werden, so dass Blut aus den Knochen austritt. Von einem minimal invasiven Eingriff kann daher nicht die Rede sein.

Einen gewissen Ansatz in die Richtung der möglichst minimalen Knochenresektion zeigt die DE 94 02 828 U1. Darin ist ein künstliches Hüftgelenk beschrieben, welches aus einer kugelförmigen Kappe und einer zweiteiligen Kunstpfanne besteht. Die Kappe wird als Gelenkersatz des Hüftkopfes eingesetzt. Hierzu ist ein sphärisches Abfräsen des knöchernen Hüftkopfes notwendig.

Auch für den Einsatz der Kunstpfanne in Form einer Metallschale ist die Ausfräsung des natürlichen Acetabulums notwendig. Insgesamt also wird bei der Implantation dieses Hüftgelenk weiterhin der Knochen mit Fräsern bearbeitet, wenn auch in einem geringeren Maße als bei der Implantation der übrigen erwähnten Hüftgelenke. In jedem Falle wird Knochenmaterial angefrischt, was zu Blutungen führt, die unter anderem negativen Einfluss auf die Langzeitfixaktion haben können.

Vor diesem Hindergrund ist es die Aufgabe der vorliegenden Erfindung, ein eingangs erwähntes Set zur Erstellung eines Resurfacing-Hüftimplantates anzugeben, welches die größtmögliche Flexibilität hinsichtlich eines Langzeiterhaltes des Implantates in situ ermöglicht, also eine weitergehende endoprothetische Versorgung hilft hinauszuzögern.

Gelöst wird diese Aufgabe mit einem Set mit den Merkmalen gemäß dem Anspruch 1. Vorteilhafte Weiterbildungen ergeben sich aus den Unteransprüchen.

Demgemäß weist das Set eine 1 bis 1,5 mm dicke metallische Schale zum zementierten Einsatz in das natürliche, lediglich entknorpelte Acetabulum, deren Aussenkonturen dem natürlichen Acetabulum nachgebildet sind, sowie eine 1 bis 1,5 mm dicke metallische Kappe zum zementierten Aufsatz auf den natürlichen, lediglich entknorpelten Hüftgelenkkopf, deren Form jene des Hüftgelenkkopfes nachgebildet ist, und darüber hinaus ein in die Acetabulums-Schale einsetzbares Inlay mit einer Materialstärke zwischen 2 bis 5 mm als Gleitpartner für die Hüftgelenkskopf-Kappe auf.

Sowohl das knöcherne Acetabulum als auch der knöcherne Hüftgelenkskopf werden nicht mit einem Formfräser bearbeitet, wie dies bei der Implantation eines Hüftgelenkstotalersatzimplantates der Fall ist. Acetabulum und Gelenkkopf werden lediglich von Knorpel und Bindegewebe befreit, das heißt entknorpelt. Sodann wird die Schale für das Acetabulum rückseitig mit Zement in das Acetabulum gesetzt, wo der Zement abbindet und der Schale so einen sicheren Sitz im Acetabulum bietet. Entsprechend wird die Kappe mit Zement über den Gelenkkopf gezogen und dort durch die Abbindung des Zementes fixiert. Die Materialstärke der Schale und der Kappe liegen zwischen etwa 1 und 1,5 mm. Die Stärke des Inlays liegt zwischen 2 und 5 mm. Diese Materialstärke wird als Ausgleichsstärke angesehen und zwar für das entfernte Knorpel- und Bindegewebe. Die Verwendung des Inlays bietet die Möglichkeit, es nach einem etwaigen Abrieb durch ein neues Inlay zu ersetzen und so die Standzeit des Resurfacing-Implantates deutlich zu verlängern, um so einen massiveren Eingriff, beispielsweise durch Implantation eines Kappenimplantates (DE-C-102 18 801, DE 94 028 28, DE-A-20 39 731) mit Knochenzurichtung hinauszuzögern, falls er denn notwendig werden sollte. Im Gegensatz zu den Implantaten gemäß der vorerwähnten Druckschriften stellt das erfindungsgemäße Implantat

kein Ersatzimplantat, sondern vielmehr ein Rekonstruktionsimplantat dar. Im Falle eines Ersatzes stehen vier weitere Stufen der endoprothetischen Versorgung zur Verfügung, nämlich zunächst durch das Kappenimplantat, gefolgt von der Kurzstielendoprothese gemäß der EP-B-0 878 176. Sodann kann in der nächsten Stufe eine metadiaphysäre Endoprothese gemäß der nicht vorveröffentlichten deutschen Patentanmeldung 10 2004 051 431 zur Anwendung kommen. Am Ende der endoprothetischen Versorgung steht schließlich die Versorgung des Patienten mit einer der bekannten Langstielendoprothesen. Rechnet man mit einer Standzeit von durchschnittlich 15 Jahren der Endoprothesen in jeder Stufe, so ergibt sich theoretisch mit dem Einsatz des erfindungsgemäßen Resurfacing-Hüftimplantates eine gesamte prothetische Versorgungszeit von 75 Jahren. Dieses Versorgungssystem bietet also ausreichende Reserven für den praktischen Einsatz.

Das Inlay des Sets ist bevorzugt in die Acetabulums-Schale in einen konischen Klemmsitz bringbar. Dieser ist lösbar, so dass das Inlay ausgewechselt werden kann.

Wenn die Hüftgelenkskopf-Kappe gemäß einer vorteilhaften Weiterbildung eine keramisierte Oberfläche als Artikulationsfläche aufweist, besteht das Inlay bevorzugt aus einem keramischen Kompositwerkstoff, wie er beispielsweise bekannt ist aus der EP-B-0 502 082. Diese Materialkombination ist besonders abriebsarm.

Im Polbereich der Acetabulums-Schale kann vorteilhaft eine Öffnung vorgesehen sein, durch welche eine Positionierhilfe setzbar ist. Bei der Positionierhilfe kann es sich beispielsweise um eine Blindschraube handeln, die nach dem Einbringen dauerhaft in den Boden des Acetabulums greift. Sie führt Führungsaufgaben aus, die eine exakte Positionierung der Schale im Acetabulum ermöglicht.

Besonders bevorzugt wird eine Weiterbildung, bei der im Inneren der Hüftgelenkskopf-Kappe wenigstens zwei Antirotationselemente in das Kappeninnere hineinragen. Dies erhöht die Sicherheit des Sitzes der Kappe auf dem Hüftgelenkskopf. Die Antirotationselemente ragen nach der Implantation in den Knochen des Hüftgelenkskopfes hinein und verhindern so ein Drehen der Kappe auf dem Gelenkkopf.

Gemäß einer konkreten Ausführungsform sind die Antirotationselemente schildförmig ausgebildet. Alternativ können sie zapfenförmig ausgebildet sein.

Die für den Einsatz des Implantates erforderliche Operation ist für den Patienten äußert schonend, da sie nur von geringer Dauer ist. Geübten Operateuren gelingt es, das Implantat innerhalb von ca. 35 Minuten zu platzieren. Nur ein kleiner Schnitt ist hierfür von Nöten, der innerhalb weniger Tage verheilt ist.

Die Erfindung wird anhand der Zeichnungsfiguren beispielhaft näher erläutert. Hierbei zeigt:
- Fig. 1: schematisch das aus dem Set zusammengestellte Hüftimplantat mit der im Acetabulum fixierten Schale sowie auf dem Gelenkkopf platzierten Kappe
- Fig. 2: die Einzelteile des erfindungsgemäßen Sets,
- Figur 3: eine Ansicht in das Innere einer Ausführungsform der Hüftgelenkskopf-Kappe und
- Figur 4: eine Ansicht in das Kappeninnere einer weiteren Ausführungsform der Hüftgelenkskopf-Kappe.

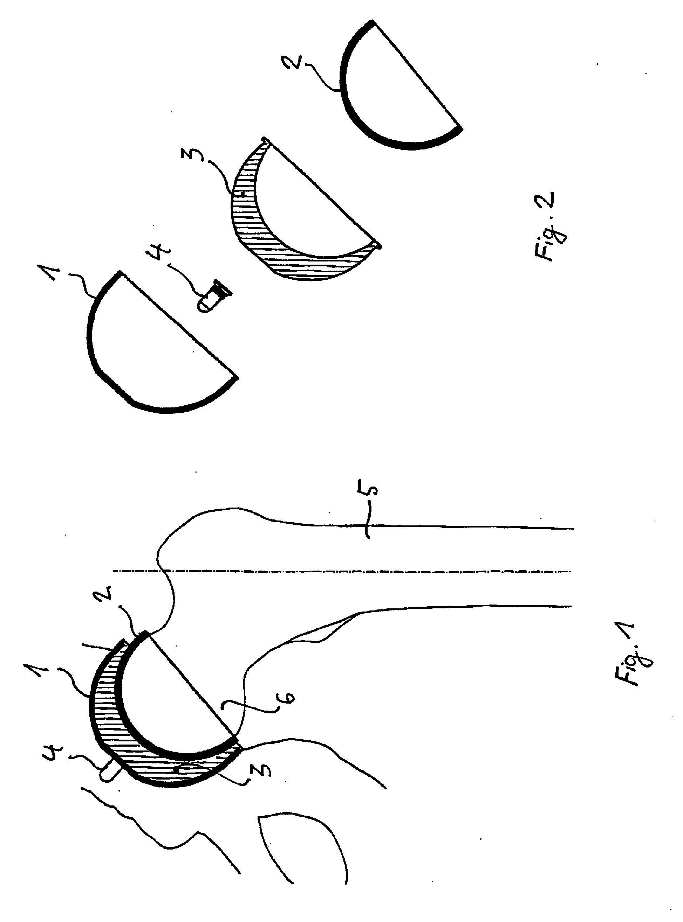

Figur 1 veranschaulicht schematisch, wie das Hüftimplantat, das aus dem Set erstellt worden ist, implantiert ist. Auf dem Hüftgelenkskopf 6 am Femur 5 ist die metallische Kappe 2 gesetzt. Sie wird dort mittels einer dünnen Zementschicht im Inneren der Kappe fixiert.

In das natürliche Acetabulum 7 ist die metallische Schale 1 gesetzt und dort mit einer dünnen Zementschicht fixiert. Der verwendete Zement ist sehr dünnflüssig.

In die Schale 1 ist das Inlay 3 gesetzt, welches den Gleitpartner für die metallische Kappe 2 bildet. In den Beckenknochen hinein ragt eine Positionierhilfe 4, beispielsweise in Form einer Blindschraube, die durch eine Öffnung im Polbereich der Schale 1 eingesetzt ist und von dort in den Beckenknochen ragt.

Bei der Erstellung des Resurfacing-Hüftimplanates wird zunächst die Positionierhilfe 4 durch die Öffnung am Polbereich der Schale 1 gesetzt (Fig. 2). So dann erfolgt der Einsatz des Inlays 3 in die Schale 1. Das Inlay 3 sitzt in der Schale 1 in einem (konischen) Klemmsitz und ist auswechselbar. Eine hierzu ausgewählte Kappe 2 wird so dann für den Aufsatz auf den Hüftgelenkskopf ausgewählt.

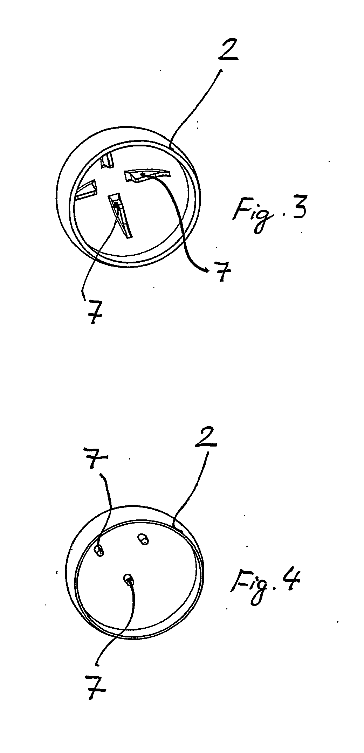

Figur 3 zeigt eine perspektivische Ansicht in das Innere der Hüftgelenkskopf-Kappe 2. Deutlich erkennbar sind in den dargestellten Ausführungsbeispielen vier Antirotationselemente 7 in Form von Schilden. Diese greifen nach dem Aufsetzen der Kappe 2 auf den Hüftgelenkskopf in das knöcherne Material des Hüftgelenkskopfes hinein und sichern den Sitz der Kappe 2 auf dem Hüftgelenkskopf gegen eine Drehung. Hierdurch wird der Sitz der Kappe 2 auf dem Hüftgelenkskopf nochmals stabilisiert.

Eine ähnliche Ansicht in das Innere der Kappe 2 gemäß einer weiteren Ausführungsform zeigt Figur 4. Hier sind drei Antirotationselemente 7 in Form von Zapfen abgebildet. Auch diese Zapfen 7 greifen nach dem Aufsetzen der Kappe auf den Hüftgelenkskopf in das knöcherne Material des Hüftgelenkskopfes und sichern so die Kappe 2 gegen eine Rotation auf dem Hüftgelenkskopf.

## Patentansprüche

1. Set zur Erstellung eines Resurfacing-Hüftimplantates, aufweisend eine 1 bis 1,5 mm dicke metallische Schale (1) zum zementierten Einsatz in das natürliche, lediglich entknorpelte Acetabulum sowie eine 1 bis 1,5 mm dicke metallische Kappe (2) zum zementierten Aufsatz auf den natürlichen, lediglich entknorpelten Hüftgelenkskopf und darüber hinaus ein in die Acetabulums-Schale (1) einsetzbares Inlay (3) mit einer Materialstärke zwischen 2 bis 5 mm als Gleitpartner für die Hüftgelenkskopf-Kappe (2).

2. Set nach Anspruch 1, bei dem das Inlay (3) in die Acetabulums-Schate (1) in einen konischen Klemmsitz bringbar ist.

3. Set nach Anspruch 1 oder 2, bei dem das Inlay (3) aus hochverdichtetem Polyethylen besteht.

4. Set nach Anspruch 1 oder 2, bei dem die Hüftgelenkskopf-Kappe (2) eine keramisierte Oberfläche als Artikulationsfläche aufweist und das Inlay (3) aus einem keramischen Kompositwerkstoff besteht.

5. Set nach einem der Ansprüche 1 bis 4. bei dem im Polbereich der Acetabulums-Schale (1) eine Öffnung vorgesehen ist, durch welche eine Positionierhilfe (4) setzbar ist.

## Claims

1. Set for constructing a resurfacing hip implant having a 1 to 1.5 mm thick metallic cup (1) for cemented insertion into the natural acetabulum which is simply devoid of cartilage and a 1 to 1.5 mm thick metallic cap (2) for cemented mounting on the natural hip condyle which is simply devoid of cartilage and furthermore an inlay (3) which can be inserted into the acetabulum cup (1) and having a material thickness between 2 to 5 mm as a sliding partner for the hip condyle cap (2).

2. Set according to claim 1, in which the inlay (3) can be introduced into the acetabulum cup (1) in a conical press fit.

3. Set according to claim 1 or 2, in which the inlay (3) consists of high-density polyethylene.

4. Set according to claim 1 or 2, in which the hip condyle cap (2) has a ceramic surface as the articulation surface and the inlay (3) consists of a ceramic composite material.

5. Set according to one of claims 1 to 4, in which an opening, through which a positioning aid (4) can be placed, is provided in the pole region of the acetabulum cup (1).

## Revendications

1. Ensemble pour la réalisation d'un implant de resurfaçage de la hanche, comportant une coque (1) métallique de 1 à 1,5 mm d'épaisseur, destinée à être mise en place par cimentage dans la cavité cotyloïde naturelle, dont seul le cartilage a été excisé, ainsi qu'une calotte (2) métallique de 1 à 1,5 mm d'épaisseur, destinée à être posée par cimentage sur la tête fémorale naturelle, dont seul le cartilage a été excisé, et, en outre, une incrustation (3), apte à être mise en place dans la coque (1) de cavité cotyloïde et ayant une épaisseur de matériau entre 2 à 5 mm, formant un élément de glissement pour la calotte (2) de tête fémorale.

2. Ensemble selon la revendication 1, dans lequel l'incrustation (3) peut être amenée dans la coque (1) de cavité cotyloïde dans un ajustement serré conique.

3. Ensemble selon la revendication 1 ou 2, dans lequel l'incrustation (3) est réalisée en polyéthylène à haute densité.

4. Ensemble selon la revendication 1 ou 2, dans lequel la calotte (2) de tête fémorale comporte une surface supérieure céramisée formant une surface d'articulation, et l'incrustation (3) est réalisée dans un matériau composite céramique.

5. Ensemble selon l'une quelconque des revendications 1 à 4, dans lequel il est prévu de réaliser dans la zone polaire de la coque (1) de cavité cotyloïde un orifice, à travers lequel peut être posé un élément d'aide au positionnement (4).
